# EUROPEAN PATENT APPLICATION

(11) **EP 3 936 119 A1**
(43) Date of publication of application: **12.01.2022**
(21) Application number: 20766019.2
(22) Date of filing: 03.03.2020
(51) Int. Cl.: A61K 9/20, A61K 9/48, A61K 31/44, A61P 7/06

(54) **PHARMACEUTICAL COMPOSITION OF PROLYL HYDROXYLASE INHIBITOR AND PREPARATION METHOD THEREFOR**

(30) Priority: 04.03.2019 CN 201910160454
(71) Applicant: Jiangsu Hengrui Medicine Co., Ltd., Lianyungang, Jiangsu 222047 (CN); Suzhou Suncadia Biopharmaceuticals Co., Ltd., Suzhou, Jiangsu 215126 (CN)
(72) Inventor: YANG, Zhiliang, Lianyungang, Jiangsu 222047 (CN); SHI, Lei, Lianyungang, Jiangsu 222047 (CN); WANG, Luying, Lianyungang, Jiangsu 222047 (CN)
(74) Representative: Potter Clarkson
(86) International application number: PCT/CN2020/077560
(87) International publication number: WO 2020/177681

(57) **Abstract**

The present application provides a pharmaceutical composition of a prolyl hydroxylase inhibitor and a preparation method therefor. In particular, the present application provides a composition comprising a compound represented by formula (I) or a pharmacologically acceptable salt thereof and at least one water-soluble filler. The composition provided by the present application has a rapid dissolution rate and good stability.

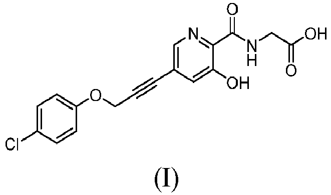

## Description

### TECHNICAL FIELD

The present application belongs to the field of medicine, and relates to a pharmaceutical composition of a prolyl hydroxylase inhibitor.

### BACKGROUND

Anemia generally refers to any abnormality in hemoglobin or red blood cells that leads to reduced oxygen levels in the blood. Anemia can also develop in association with chronic diseases, such as chronic infection, neoplastic diseases, chronic inflammation, including disorders of consequent inflammatory suppression of marrow, etc. Anemia of chronic disease, for example anemia in chronic kidney disease, is one of the most common syndromes in medicine. The main cause of anemia in chronic kidney disease is insufficient secretion of erythropoietin (EPO) *(*Nephrol Dial Transplant 17 (2002)2-7). The insufficient secretion of EPO can hinder the production of red blood cells, resulting in the occurrence of anemia. The expression and secretion of EPO are regulated by the transcription factor hypoxia inducible factor (HIF). The HIF protein with complete transcription function is composed of two subunits HIF-α and HIF-β, of which HIF-α is regulated by prolyl hydroxylase (PHD) that can hydroxylate HIF-α to promote its degradation. Inside the human body, prolyl hydroxylase 2 (PHD2) is the most dominant subtype that regulates HIF levels (Journal of Medicinal Chemistry 56 (2013)9369-9402). When the activity of prolyl hydroxylase (PHD) *in vivo* is inhibited, the HIF-α subunit can be stabilized *in vivo,* so that it enters the nucleus, and binds to the HIF-β subunit in the nucleus to form a stable HIF dimer. The dimer further causes the expression of downstream genes, thereby promoting the expression and secretion of EPO. Therefore, the inhibition of activity of prolyl hydroxylase can increase HIF-α level and promote the production of EPO, thereby promoting the maturation of red blood cells, enhancing the capacity of blood in delivering oxygen, and improving anemia or ischemic symptoms.

WO2017059623 discloses a novel class of alkynyl pyridines prolyl hydroxylase inhibitors. Among them, the compound of formula (I), whose chemical name is 2-(3-hydroxy-5-(3-*p*-chlorophenoxypropyn-1-yl))picolinamido acetic acid, shows an excellent inhibition effect on prolyl hydroxylase, and is a potential new drug for treating chronic anemia.

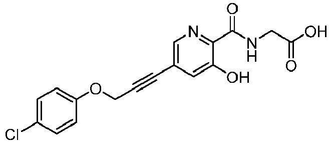

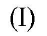

In order to meet the medication needs of patients, the compound of formula (I) needs to be prepared into a suitable formulation. Regarding to a solid formulation, it needs to have good dissolution and stability.

### SUMMARY OF THE INVENTION

The present application provides a pharmaceutical composition with a good stability and rapid dissolution. The process for preparing the pharmaceutical composition is simple and is suitable for large-scale production.

The present application provides a pharmaceutical composition, comprising a compound of formula (I) or a pharmacologically acceptable salt thereof and at least one water-soluble filler,

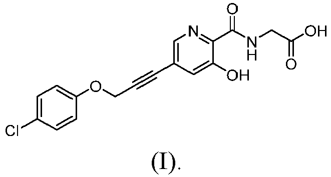

The water-soluble filler described in the present application can be a sugar alcohol, and preferably one or more of lactose, glucose, sucrose, mannitol and sorbitol.

In some embodiments, the water-soluble filler in the present application is lactose or mannitol, and preferably lactose.

The water-soluble filler in the present application is present in an amount of 15% to 95%, preferably 30% to 80%, and more preferably 40% to 75% by weight, relative to the total weight of the composition.

The pharmaceutical composition provided in the present application can comprise at least one additional filler, such as starch, pregelatinized starch, dextrin, microcrystalline cellulose, calcium hydrogen phosphate, and preferably microcrystalline cellulose.

The additional filler in the pharmaceutical composition provided in the present application is present in an amount of 1% to 80%, preferably 10% to 50%, and most preferably 15% to 30% by weight, relative to the total weight of the composition.

In some embodiments, the water-soluble filler in the composition is lactose, and the additional filler is microcrystalline cellulose.

The pharmaceutical composition provided in the present application further comprises a disintegrant, wherein the disintegrant is one or more selected from the group consisting of croscarmellose sodium, sodium carboxymethyl starch, low-substituted hydroxypropyl cellulose and crospovidone, and preferably croscarmellose sodium.

The disintegrant in the pharmaceutical composition provided in the present application is present in an amount of 0.1% to 20%, preferably 1% to 10%, and more preferably 2% to 5% by weight, relative to the total weight of the composition.

The pharmaceutical composition provided in the present application further comprises a lubricant, wherein the lubricant is one or more selected from the group consisting of talc, magnesium stearate, zinc stearate, sodium stearyl fumarate, glyceryl behenate, sodium lauryl sulfate and hydrogenated vegetable oil, and is present in an amount of 0.1% to 5%, preferably 0.1% to 3%, and most preferably 1% to 2% by weight, relative to the total weight of the composition. Specifically, it can be 0.1%, 0.2%, 0.3%, 0.4%, 0.5%, 0.6%, 0.7%, 0.8%, 0.9%, 1%, 1.5%, 2%, 2.5%, 3%, 3.5%, 4%, 4.5%, 5%.

The pharmaceutical composition provided in the present application can further comprise a glidant, wherein the glidant is selected from the group consisting of silicon dioxide, corn starch, siliciidoxydum and talc, and preferably siliciidoxydum, and is present in an amount of 0.1% to 10%, preferably 0.5% to 5%, and most preferably 0.5% to 2.0% by weight, relative to the total weight of the composition.

The active ingredient the compound of formula (I) or the pharmacologically acceptable salt thereof in the pharmaceutical composition provided in the present application is present in an amount of 0.1% to 70%, preferably 5% to 50%, and most preferably 15% to 35% by weight, relative to the total weight of the composition.

In the pharmaceutical composition provided in the present application, the amount of the compound of formula (I) or the pharmacologically acceptable salt thereof in an unit dosage form is 1 to 1000 mg, and preferably 5 to 500 mg. Specifically, the amount can be 10 mg, 15 mg, 20 mg, 25 mg, 30 mg, 35 mg, 40 mg, 45 mg, 50 mg, 55 mg, 60 mg, 65 mg, 70 mg, 75 mg, 80 mg, 85 mg, 90 mg, 95 mg, 100 mg, 105 mg, 110 mg, 115 mg, 120 mg, 125 mg, 130 mg, 135 mg, 140 mg, 145 mg, 150 mg, 155 mg, 160 mg, 165 mg, 170 mg, 175 mg, 180 mg, 185 mg, 190 mg, 195 mg, 200 mg, 205 mg, 210 mg, 215 mg, 220 mg, 225 mg, 230 mg, 235 mg, 240 mg, 245 mg, 250 mg, 255 mg, 260 mg, 265 mg, 270 mg, 275 mg, 280 mg, 285 mg, 290 mg, 295 mg, 300 mg, 305 mg, 310 mg, 315 mg, 320 mg, 325 mg, 330 mg, 335 mg, 340 mg, 345 mg, 350 mg, 355 mg, 360 mg, 365 mg, 370 mg, 375 mg, 380 mg, 385 mg, 390 mg, 395 mg, 400 mg, 405 mg, 410 mg, 415 mg, 420 mg, 425 mg, 430 mg, 435 mg, 440 mg, 445 mg, 450 mg, 455 mg, 460 mg, 465 mg, 470 mg, 475 mg, 480 mg, 485 mg, 490 mg, 495 mg, 500 mg, and preferably 25 mg, 50 mg, 75 mg, 100 mg, 125 mg, 150 mg, 175 mg, 200 mg, 225 mg, 250 mg, 275 mg, 300 mg, 325 mg, 350 mg, 375 mg, 400 mg, 425 mg, 450 mg, 475 mg, or 500 mg.

In the pharmaceutical composition provided in the present application, the d0.9 particle size of the compound of formula (I) or the pharmacologically acceptable salt thereof is less than 200 µm, and preferably less than 150 µm.

The present application provides a pharmaceutical composition, comprising the following components:
15% to 35% of the compound of formula (I) or the pharmacologically acceptable salt thereof with a d0.9 particle size distribution of less than 150 µm;
40% to 75% of a sugar alcohol filler, and preferably lactose or mannitol; optionally 10% to 50% of microcrystalline cellulose;
0.1% to 20% of a disintegrant, wherein the disintegrant is one or more selected from the group consisting of croscarmellose sodium, sodium carboxymethyl starch, low-substituted hydroxypropyl cellulose and crospovidone;
0.1% to 5% of a lubricant, wherein the lubricant is one or more selected from the group consisting of talc, magnesium stearate, zinc stearate, sodium stearyl fumarate, glyceryl behenate, sodium lauryl sulfate and hydrogenated vegetable oil; and
0.1% to 10% of a glidant, wherein the glidant is one or more selected from the group consisting of silicon dioxide, corn starch, siliciidoxydum and talc.

The pharmaceutical composition provided in the present application can be a tablet or a capsule.

In some embodiments, the composition provided in the present application comprises a coating layer, wherein the coating material is a gastric-soluble coating, and can be specifically one or more selected from the group consisting of polyvinylacetal diethylaminoacetate, Eudragit E series, hydroxypropyl dibutyl cellulose acetate ether, methyl vinyl pyridine, methacrylate and methacrylic acid copolymer, hydroxypropyl cellulose, hydroxypropyl methyl cellulose and Opadry^{®} 85G68918. The weight gain of the coating layer accounts for 1% to 10%, and preferably 2% to 5% of the total weight of the tablet core.

The pharmaceutical composition of the present application can be prepared by a conventional method in the art. The active ingredient the compound of formula (I) or the pharmacologically acceptable salt thereof is mixed with the water-soluble filler to obtain a mixture, and the mixture is further subjected to wet granulation, dry granulation or powder direct compression, and preferably dry granulation or powder direct compression.

In some embodiments, the pharmaceutical composition provided in the present application is a tablet, and the preparation method thereof further comprises a coating step, and the coating agent in the coating step is a gastric-soluble coating.

The pharmaceutical composition provided in the present application can be used in the preparation of a medicament for treating a prolyl hydroxylase-mediated disease, and preferably anemia.

The pharmaceutical composition provided in the present application has a good drug dissolution rate due to the presence of the water-soluble filler. The dissolution rate of the pharmaceutical composition is ≥75% in 45 minutes, preferably ≥80% in 45 minutes, and most preferably ≥90% in 45 minutes, determined according to the Second Method of General Rule 0931 of Volume IV of Chinese Pharmacopoeia 2015 Edition, using 900 ml of phosphate buffer (pH 5.8) (0.5% Tween 80) as a dissolution medium at a speed of 75 rpm.

In another aspect, the pharmaceutical composition provided in the present application has a good stability. The total impurities are less than 2% after the pharmaceutical composition has been left to stand in an open condition, at a temperature of 60°C, or at a temperature of 40°C and a relative humidity of 75% for 14 days or 30 days.

### DESCRPTION OF THE DRAWING

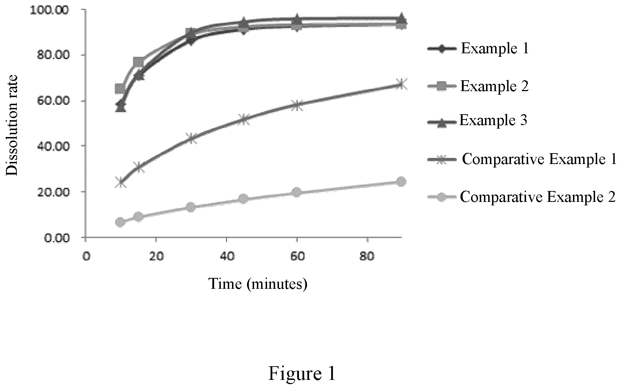

Figure 1. Dissolution profiles of Examples 1 to 3 and Comparative Examples 1 and 2.

### DETAILED DESCRIPTION

The present invention is further described in detail by the following examples and experimental examples. These examples and experimental examples are for illustrative purposes only, and are not intended to limit the scope of the present invention.

### Examples 1 to 3, and Comparative Examples 1 and 2

The materials were weighed accurately. At the beginning, a small amount of lactose (or mannitol or microcrystalline cellulose or anhydrous calcium hydrogen phosphate) was passed through a 40 mesh sieve. Then the compound of formula (I) (active ingredient) and the remaining lactose (or mannitol or microcrystalline cellulose or anhydrous calcium hydrogen phosphate) were mixed and passed through the sieve together. Then microcrystalline cellulose, anhydrous calcium hydrogen phosphate, silicon dioxide and the like were passed through the sieve. The mixture was placed in a suitable mixing bottle and mixed for 15 minutes. Magnesium stearate was added, and the mixture was further mixed for 5 minutes. The mixture was weighed and compressed by a single-punch tableting machine equipped with a ϕ6mm shallow concave circular punch, the target tablet weight was 100 mg (95 to 105 mg), and the target hardness was 60 N (40 to 80 N). According to the above preparation method, the tablets of Examples 1 to 3 and Comparative Examples 1 and 2 were prepared.

**Table 1**

| Ingredients | Example 1 | Example 2 | Example 3 | Comparative Example 1 | Comparative Example 2 |
|---|---|---|---|---|---|
| Active ingredient | 2.5 | 2.5 | 2.5 | 2.5 | 2.5 |
| Lactose | 5 | 0 | 7.15 | 0 | 0 |
| Mannitol | 0 | 5 | 0 | 0 | 0 |
| Microcrystalline cellulose | 2.15 | 2.15 | 0 | 2.15 | 0 |
| Anhydrous calcium hydrogen phosphate | 0 | 0 | 0 | 5 | 7.15 |
| Croscarmellose sodium | 0.2 | 0.2 | 0.2 | 0.2 | 0.2 |
| Siliciidoxydum | 0.05 | 0.05 | 0.05 | 0.05 | 0.05 |
| Magnesium stearate | 0.1 | 0.1 | 0.1 | 0.1 | 0.1 |
| Total | 10g | 10g | 10g | 10g | 10g |

### Examples 4 to 6, and Comparative Examples 3 and 4

According to the prescription and preparation method of Example 1, the tablets of Examples 4 to 6 and Comparative Examples 3 and 4 in Table 2 were obtained by controlling the particle size of the active ingredient. The effect of different particle size of the API on product dissolution was investigated.

**Table 2**

| | Example 4 | Example 5 | Example 6 | Comparative Example 3 | Comparative Example 4 |
|---|---|---|---|---|---|
| d0.9 particle size distribution of the active ingredient (µm) | 150 | 49.9 | 28.9 | 466 | 672 |

### Experimental Example 1. Effect of different prescriptions on product dissolution

Test solution: According to the dissolution and release test (the Second Method of General Rule 0931 of Volume IV of Chinese Pharmacopoeia 2015 Edition), three tablets were added to 900 ml of phosphate buffer (pH 5.8) (0.5% Tween 80) as a dissolution medium and stirred at a speed of 75 rpm. 2 ml of the solution was collected at 10, 15, 30, 45, 60 and 90 minutes, and filtered through a 0.45 µm filter membrane to obtain the test solution.

Reference solution: An appropriate amount of the active ingredient reference was dissolved in the dissolution medium, and diluted quantitatively to obtain the reference solution. The test solution and reference solution were injected into a liquid chromatograph to record the chromatogram and measure the peak area, respectively. The dissolution amount of each tablet at different times was calculated.

The dissolution rates of Examples 1 to 3 and Comparative Examples 1 and 2 are shown in Table 3.

**Table 3**

| Experiment No. | Cumulative dissolution (%) | | | | | |
|---|---|---|---|---|---|---|
| | 10 min | 15 min | 30 min | 45 min | 60 min | 90 min |
| Example 1 | 58.54 | 70.97 | 86.37 | 91.24 | 92.58 | 93.45 |
| Example 2 | 64.73 | 76.82 | 89.06 | 92.24 | 93.14 | 93.50 |
| Example 3 | 57.67 | 71.71 | 89.71 | 94.49 | 95.83 | 96.05 |
| Comparative Example 1 | 24.20 | 30.75 | 43.30 | 51.78 | 58.17 | 67.05 |
| Comparative Example 2 | 6.78 | 8.96 | 13.23 | 16.63 | 19.50 | 24.43 |

The results indicate that the Examples that comprise mannitol or lactose in the prescription have rapid active ingredient dissolution, whereas the Comparative Examples that do not comprise mannitol or lactose have slow and incomplete dissolution. The dissolution profiles are shown in Figure 1.

The dissolution rates of Examples 4 to 6 and Comparative Examples 3 and 4 are shown in Table 4.

**Table 4**

| Experiment No. | Cumulative dissolution (%) | | | | | |
|---|---|---|---|---|---|---|
| | 10 min | 15 min | 30 min | 45 min | 60 min | 90 min |
| Example 4 | 42.26 | 54.20 | 76.37 | 87.95 | 93.69 | 97.44 |
| Example 5 | 55.85 | 69.04 | 86.77 | 92.04 | 93.34 | 93.98 |
| Example 6 | 58.82 | 71.35 | 86.21 | 89.59 | 90.30 | 90.46 |
| Comparative Example 3 | 25.85 | 35.49 | 58.00 | 73.99 | 85.32 | 97.90 |
| Comparative Example 4 | 12.70 | 17.97 | 30.82 | 41.29 | 49.99 | 63.76 |

The experiment results indicate that when the d0.9 particle size distribution of the active ingredient in the prescription is greater than 200 µm, the active ingredient dissolution is slow and incomplete; when d0.9 the particle size distribution of the active ingredient is less than 200 µm, the dissolution rate tends to gradually become rapid as the particle size decreases.

### Experimental Example 2: Stability study

Tablets of Examples 1 to 3 and Comparative Examples 1 and 2 were left to stand in an open condition, at a temperature of 60°C, or at a temperature of 40°C and a relative humidity of 75% for 14 days or 30 days, respectively. The formation of total impurities was determined by HPLC method. The results show that all prescriptions are stable under high temperature and high humidity conditions, and there is no obvious difference. The experiment results are shown in Table 5.

**Table 5**

| Experiment No. | Condition | Main ingredient content (%) | Total impurities (%) |
|---|---|---|---|
| Example 1 | 0d | 98.63 | 1.37 |
| | 14d, 60°C | 98.51 | 1.49 |
| | 14d, 40°C, 75%RH | 98.69 | 1.31 |
| | 30d, 60°C | 98.53 | 1.47 |
| | 30d, 40°C, 75%RH | 98.6 | 1.40 |
| Example 2 | 0d | 98.51 | 1.49 |
| | 14d, 60°C | 98.6 | 1.40 |
| | 14d, 40°C, 75%RH | 98.61 | 1.39 |
| | 30d, 60°C | 98.42 | 1.58 |
| | 30d, 40°C, 75%RH | 98.6 | 1.40 |
| Example 3 | 0d | 98.5 | 1.50 |
| | 14d, 60°C | 98.5 | 1.50 |
| | 14d, 40°C, 75%RH | 98.63 | 1.37 |
| | 30d, 60°C | 98.46 | 1.54 |
| | 30d, 40°C, 75%RH | 98.63 | 1.37 |
| Comparative Example 1 | 0d | 98.52 | 1.48 |
| | 14d, 60°C | 98.46 | 1.54 |
| | 14d, 40°C, 75%RH | 98.72 | 1.28 |
| | 30d, 60°C | 98.41 | 1.59 |
| | 30d, 40°C, 75%RH | 98.64 | 1.36 |
| Comparative Example 2 | 0d | 98.62 | 1.38 |
| | 14d, 60°C | 98.53 | 1.36 |
| | 14d, 40°C, 75%RH | 98.72 | 1.36 |
| | 30d, 60°C | 98.33 | 1.36 |
| | 30d, 40°C, 75%RH | 98.64 | 1.36 |

## Claims

1. A pharmaceutical composition, comprising a compound of formula (I) or a pharmacologically acceptable salt thereof and at least one water-soluble filler,

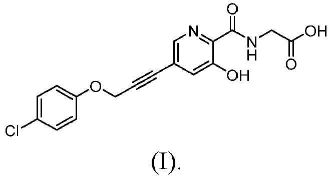

2. The pharmaceutical composition according to claim 1, wherein the water-soluble filler is a sugar alcohol, preferably one or more of lactose, glucose, sucrose, mannitol and sorbitol, and most preferably lactose or mannitol.

3. The pharmaceutical composition according to claim 1, wherein the water-soluble filler is present in an amount of 15% to 95%, preferably 30% to 80%, and more preferably 40% to 75% by weight, relative to the total weight of the composition.

4. The pharmaceutical composition according to any one of claims 1 to 3, further comprising at least one additional filler, wherein the additional filler is selected from the group consisting of starch, pregelatinized starch, dextrin, microcrystalline cellulose and calcium hydrogen phosphate, and preferably microcrystalline cellulose.

5. The pharmaceutical composition according to claim 4, wherein the additional filler is present in an amount of 1% to 80%, preferably 10% to 50%, and most preferably 15% to 30% by weight, relative to the total weight of the composition.

6. The pharmaceutical composition according to any one of claims 1 to 5, further comprising a disintegrant, wherein the disintegrant is one or more selected from the group consisting of croscarmellose sodium, sodium carboxymethyl starch, low-substituted hydroxypropyl cellulose and crospovidone, and preferably croscarmellose sodium.

7. The pharmaceutical composition according to claim 6, wherein the disintegrant is present in an amount of 0.1% to 20%, preferably 1% to 10%, and more preferably 2% to 5% by weight, relative to the total weight of the composition.

8. The pharmaceutical composition according to any one of claims 1 to 7, further comprising a lubricant, wherein the lubricant is one or more selected from the group consisting of talc, magnesium stearate, zinc stearate, sodium stearyl fumarate, glyceryl behenate, sodium lauryl sulfate and hydrogenated vegetable oil, and is present in an amount of 0.1% to 5%, and preferably 1% to 3% by weight, relative to the total weight of the composition.

9. The pharmaceutical composition according to any one of claims 1 to 8, wherein the compound of formula (I) or the pharmacologically acceptable salt thereof is present in an amount of 0.1% to 70%, preferably 5% to 50%, and most preferably 15% to 35% by weight, relative to the total weight of the composition.

10. The pharmaceutical composition according to any one of claims 1 to 9, further comprising a glidant, wherein the glidant is one or more selected from the group consisting of silicon dioxide, corn starch, siliciidoxydum and talc, and preferably siliciidoxydum, and is present in an amount of 0.1% to 10%, preferably 0.5% to 5%, and most preferably 0.5% to 2.0% by weight, relative to the total weight of the composition.

11. The pharmaceutical composition according to any one of claims 1 to 10, wherein the amount of the compound of formula (I) or the pharmacologically acceptable salt thereof in an unit dosage form is 1 to 1000 mg, preferably 5 to 500 mg, and most preferably 25 mg, 50 mg, 100 mg, 125 mg, 150 mg, 175 mg, or 200 mg.

12. The pharmaceutical composition according to any one of claims 1 to 11, wherein the d0.9 particle size of the compound of formula (I) or the pharmacologically acceptable salt thereof is less than 200 µm, and preferably less than 150 µm, determined by a laser particle analyzer.

13. A pharmaceutical composition, comprising the following components:
15% to 35% of a compound of formula (I) or a pharmacologically acceptable salt thereof with a d0.9 particle size distribution of less than 150 µm;
40% to 75% of a sugar alcohol filler, and preferably lactose or mannitol;
optionally 10% to 50% of microcrystalline cellulose;
0.1% to 20% of a disintegrant, wherein the disintegrant is one or more selected from the group consisting of croscarmellose sodium, sodium carboxymethyl starch, low-substituted hydroxypropyl cellulose and crospovidone;
0.1% to 5% of a lubricant, wherein the lubricant is one or more selected from the group consisting of talc, magnesium stearate, zinc stearate, sodium stearyl fumarate, glyceryl behenate, sodium lauryl sulfate and hydrogenated vegetable oil; and
0.1% to 10% of a glidant, wherein the glidant is one or more selected from the group consisting of silicon dioxide, corn starch, siliciidoxydum and talc.

14. The pharmaceutical composition according to any one of claims 1 to 13, being a tablet or a capsule.

15. The pharmaceutical composition according to claim 14, further comprising a coating layer, wherein the coating material is a gastric-soluble coating, and is specifically one or more selected from the group consisting of polyvinylacetal diethylaminoacetate, Eudragit E series, hydroxypropyl dibutyl cellulose acetate ether, methyl vinyl pyridine, methacrylate and methacrylic acid copolymer, hydroxypropyl cellulose, hydroxypropyl methyl cellulose and Opadry^{®} 85G68918, and preferably Opadry^{®} 85G68918, and the weight gain of the coating layer accounts for 1% to 10%, and preferably 2% to 5% of the total weight of the tablet core.

16. The pharmaceutical composition according to any one of claims 1 to 15, wherein the dissolution rate of the composition is ≥75% in 45 minutes, preferably ≥80% in 45 minutes, and most preferably ≥90% in 45 minutes, determined according to the Second Method of General Rule 0931 of Volume IV of Chinese Pharmacopoeia 2015 Edition, using phosphate buffer as a dissolution medium at a speed of 75 rpm.

17. A method for preparing the pharmaceutical composition according to any one of claims 1 to 15, comprising a step of mixing the compound of formula (I) or the pharmacologically acceptable salt thereof with the water-soluble filler to obtain a mixture, and a step of subjecting the mixture to wet granulation, dry granulation or powder direct compression, and preferably dry granulation or powder direct compression.

18. The method according to claim 17, optionally comprising a coating step with a gastric-soluble coating material.

19. Use of the pharmaceutical composition according to any one of claims 1 to 15 in the preparation of a medicament for treating a prolyl hydroxylase-mediated disease, and preferably anemia.
